# EUROPEAN PATENT APPLICATION

(11) **EP 4 357 420 A1**
(43) Date of publication of application: **24.04.2024**
(21) Application number: 22201852.5
(22) Date of filing: 17.10.2022
(51) Int. Cl.: C09D 5/00, C09D 7/80, C12N 1/00, C12N 11/00, C12P 1/04

(54) **PROCESS FOR SUSTAINABLY COATING AUTOMOTIVE SUBSTRATES**

(71) Applicant: BASF Coatings GmbH, 48165 Münster (DE)
(72) Inventor: VAN DER AUWERA, Astrid, 48165 Münster (DE); GUTJAHR, Mark, 48165 Münster (DE); LUCHTMAN, Laura, 3037SH Rotterdam (NL)
(74) Representative: BASF IP Association

(57) **Abstract**

The present invention relates to a process for producing an at least partially colored substrate by incubating the substrate with at least one microorganism capable of producing at least one organic pigment and/or dye, applying at least one coating material to the resulting at least partially colored substrate and optionally drying and/or curing said applied coating material(s). The present invention further relates to an at least partially colored substrate produced by the inventive process.

## Description

The present invention relates to a process for producing an at least partially colored and optionally coated substrate by incubating the substrate with at least one microorganism capable of producing at least one organic pigment and/or dye, and optionally applying at least one coating material to the resulting at least partially colored substrate. The present invention further relates to an at least partially colored and optionally coated substrate obtained by said process.

### Background of the invention

A wide variety of different substrates with variable layer thicknesses are nowadays coated with coating compositions in a downstream process after production of the substrate to provide visual enhancement of the substrate and/or to provide protection of the substrate from environmental influences, such as soil, stone chipping, weathering, etc..

Such aqueous or solvent-based coating compositions normally contain pigments and/or dyes in a binder matrix which, upon curing, forms the colored coating layer. A huge majority of pigments and/or dyes currently used in such coating compositions are produced from petroleum aromatic chemistry or carbonous feedstock, thus their production contributes significantly to the global warming. Furthermore, the chemical operations necessary to purify intermediates or final products, in order to be commercialized and used, requires lots of energetic resources and also generates undesirable additional by-products.

The negative impact on the environment when using such pigments and/or dyes has stipulated the industry to use pigments and/or dyes coming from more sustainable sources, such as naturally occurring pigments present in dye plants or in the ground. This allows to improve the sustainability of such coated substrates because it significantly reduces the CO₂ generation associated with the petroleum aromatic chemistry or the carbonous feedstock used to produce pigments and/or dyes.

However, the performance of naturally occurring pigments and/or dyes must at least be comparable to pigments and/or dyes prepared by petroleum aromatic chemistry or using carbonous feedstock to ensure that the optical quality of the coated substrate achieves the high demands of industry and customers in this respect. The visual effects achieved with such pigments should be adaptable to the specific needs of the customer and should be obtainable with a wide variety of different substrates.

Thus, there remains a need to produce colored substrates, for example substrates used in the automotive industry, using sustainable pigments, i.e. pigments produced bacteria, having a high quality optical appearance. The produced coloring should be adjustable in terms of color and should be durable to environmental influences, such as UV-light, to avoid damage of the produced colored substrate during use.

### Problem

Accordingly, an object of the present invention is to provide a process for producing a colored substrate, in particular a colored automotive substrate, using sustainable pigments. The process should be suitable for a wide variety of substrates, in particular substrates commonly used in the automotive industry. The produced coloring should be adjustable in terms of color. The produced colored substrates should be overcoatable with commonly known coating materials, such as clearcoat materials, without any further pretreatment steps to improve the durability, for example with respect to environmental influences, of the colored substrate.

### Technical Solution

This objective has been solved by the subject-matter of the claims of the present application as well as by the preferred embodiments thereof disclosed in this specification, i.e., by the subject matter described herein.

A first subject-matter of the present invention is a process for producing an at least partially colored substrate (S), said method comprising
(i) inoculating at least part of a surface of the substrate (S) with at least one microorganism being capable of producing at least one organic pigment and/or dye,
(ii) immersing at least the part of the inoculated surface in a nutrient medium such that the at least one microorganism produces the at least one organic pigment and/or dye,
(iii) interrupting the pigment and/or dye producing process,
(iv) applying at least one coating material to at least part of the colored substrate and optionally drying and/or curing the applied coating material(s).

A further subject-matter of the present invention is an at least partially colored substrate (S) produced by the processes disclosed herein.

The processes disclosed herein allow to at least partially color a wide variety of substrates, such as metal substrates, plastic substrates and substrates comprising metal and plastic parts which are commonly used to fabricate vehicles, such as automotives, with sustainable pigments without the necessity to use a coating material. This allows to save resources and energy in terms of raw material need to produce the coating material and heating required for drying and/or curing of said applied coating material. Moreover, the processes disclosed herein allow to manipulate the degree of coloring and/or the resulting color by adjusting the incubation duration, thus rendering it possible to adjust the optical result to the customers' needs without extensive modifications of the microorganisms. Additionally, the processes disclosed herein allow to customize the color by selecting the microorganism producing pigments and/or dyes having the desired color. The obtained coloring can be overcoated with commonly known coating materials, such as clearcoat materials, without any further pretreatment steps, such as the application of primer coating materials. The overcoating allows to achieve a sufficient degree of protection against environmental influences, such as UV irradiation.

### Detailed description of the invention

The terms "pigment" and "dye" are known to the skilled person, e.g., from DIN 55943 (date: October 2001). A "pigment" in the sense of the present invention refers preferably to a constituent in powder or flake form which is substantially, preferably entirely, insoluble in the medium surrounding them, such as in the coating material composition, for example. Pigments are preferably colorants and/or substances which can be used as pigment on account of their magnetic, electrical and/or electromagnetic properties. Pigments differ from "fillers" preferably in their refractive index, which for pigments is ≥ 1.7. The term "filler" is known to the skilled person, from DIN 55943 (date: October 2001), for example. Pigments in general can be inorganic or organic. However, if constituent a1) is a pigment, it is an organic pigment, preferably an organic color (coloring) pigment. The term "dye" is known to the skilled person, from DIN 55943 (date: October 2001), for example. A "dye" in the sense of the present invention refers preferably to a colorant, which is substantially, preferably entirely, soluble in the medium surrounding them, such as in the coating material composition, for example. Pigments and dyes both represent colorants.

The term "nutrient medium" refers to a substance used for the cultivation, isolation, identification, or storage of microorganisms. The nutrient medium may be liquid or may be solid. Solid nutrient medium may be obtained using thickening agents, such as agar.

A filler layer (primer-surfacer layer) is describing an intermediate layer used to fill out the irregularities of the substrate, to support corrosion resistance and adhesion as well as to provide protection from mechanical exposure such as stone chipping. To allow the cured filler layer to fulfill the objectives identified above, film thicknesses of 25 to 45 micrometers are commonly used.

A primer layer is describing the first layer of a multilayer coating which is applied onto the substrate and is used to provide improved adhesion for the multilayer coating. Moreover, the primer layer can provide improved corrosion protection, for example on metallic substrates.

Drying of an applied coating material denotes the vaporization of organic solvents and/or water present in a coating composition after application, usually at a temperature elevated relative to ambient temperature, for example of 40 to 90 °C, for a period of, for example, 1 to 60 minutes. However, the intermediate drying does not give a coating film in a state ready for use either, i.e. a cured coating film as described below. Accordingly, curing of a coating film is understood to mean the conversion of such a film to the ready-to-use state, i.e. to a state in which the substrate provided with the respective coating film can be transported, stored and used as intended. More particularly, a cured coating film is no longer soft or tacky, but has been conditioned as a solid coating film which does not undergo any further significant change in its properties, such as hardness or adhesion on the substrate, even under further exposure to curing conditions as described below.

A "binder" in the context of the present invention and in accordance with DIN EN ISO 4618:2007-03 is the nonvolatile component of a coating composition, without pigments and fillers. Hereinafter, however, the expression is used principally in relation to particular physically and/or thermally curable polymers, examples being polyurethanes, polyesters, polyethers, polyureas, polyacrylates, polysiloxanes and/or copolymers of the stated polymers. A copolymer in the context of the present invention refers to polymer particles formed from different polymers. This explicitly includes both polymers bonded covalently to one another and those in which the different polymers are bound to one another by adhesion. Combinations of the two types of bonding are also covered by this definition. The nonvolatile fraction may be determined by the method described in the Examples section.

All temperatures elucidated in the context of the present invention should be understood as the temperature of the room in which the substrate or the coated substrate is located. It does not mean, therefore, that the substrate itself is required to have the temperature in question.

### A process for producing an at least partially colored substrate (S):

The process of this invention is suitable for a variety of metallic and plastic substrates as well as for substrates comprising metallic and plastic parts. The metallic or plastic substrates may be pretreated or may be coated with at least one coating layer, such as described below.

Suitable metal substrates are selected from the group comprising or consisting of steel, iron, aluminum, copper, zinc and magnesium substrates as well as substrates made of alloys of steel, iron, aluminum, copper, zinc and magnesium.

Coated and uncoated metal substrates can be pretreated in a manner known per se, i.e., for example, cleaned and/or provided with known conversion coatings. Cleaning can be effected mechanically, for example by means of wiping, grinding and/or polishing, and/or chemically by means of etching methods, such as surface etching in acid or alkali baths using, for example, hydrochloric acid or sulfuric acid, or by cleaning with organic solvents or aqueous detergents. Pretreatment by application of conversion coatings, especially by means of phosphation and/or chromation, preferably phosphation, may likewise take place. Preferably, the metallic substrates are at least conversion-coated, especially phosphated, preferably by a zinc phosphation.

Metal substrates being coated with a cured electrocoat are produced by electrophoretic application of an electrocoat material to the substrate and subsequent curing of the applied electrocoat material. The electrocoat material may be a cathodic or anodic electrocoat material, preferably a cathodic electrocoat material. Electrocoat materials are aqueous coating materials comprising anionic or cationic polymers as binders. These polymers contain functional groups which are potentially anionic, i.e. can be converted to anionic groups, for example carboxylic acid groups, or functional groups which are potentially cationic, i.e. can be converted to cationic groups, for example amino groups. The conversion to charged groups is generally achieved by the use of appropriate neutralizing agents (organic amines (anionic), organic carboxylic acids such as formic acid (cationic). The electrocoat materials generally comprise typical anticorrosion pigments. The cathodic electrocoat materials preferred in the context of the invention comprise preferably cationic polymers as binders, especially hydroxy-functional polyether amines, which preferably have aromatic structural units. These polymers are especially used in combination with blocked polyisocyanates known per se. The application of the electrocoating material proceeds by electrophoresis. For this purpose, the metallic workpiece to be coated is first dipped into a dip bath containing the coating material, and an electrical DC field is applied between the metallic workpiece and a counterelectrode. The workpiece thus functions as an electrode; the nonvolatile constituents of the electrocoat material migrate, because of the described charge of the polymers used as binders, through the electrical field to the substrate and are deposited on the substrate, forming an electrocoat film. For example, in the case of a cathodic electrocoat, the substrate is thus connected as the cathode, and the hydroxide ions which form there through water electrolysis neutralize the cationic binder, such that it is deposited on the substrate and forms an electrocoat layer. After the electrolytic application of the electrocoat material, the coated substrate is removed from the bath, optionally rinsed off with, for example, water-based rinse solutions, then optionally flashed off and/or intermediately dried, and finally cured. The dry film thickness of the cured electrocoat is, for example, 10 to 40 micrometers, preferably 15 to 25 micrometers.

Metal substrates being coated with a cured filler layer are produced by applying a filler coating composition to the substrate, optionally flashing off and/or intermediately drying said applied composition and finally curing said composition. Suitable filler coating compositions are known in the state of the art. The dry film thickness of the cured filler layer is, for example, 10 to 40 micrometers, preferably 25 to 30 micrometers.

Preferred plastic substrates are basically substrates comprising or consisting of (i) polar plastics, such as polycarbonate, polyamide, polystyrene, styrene copolymers, polyesters, polyphenylene oxides and blends of these plastics, (ii) synthetic resins such as polyurethane RIM, SMC, BMC and (iii) polyolefin substrates of the polyethylene and polypropylene type with a high rubber content, such as PP-EPDM, and surface-activated polyolefin substrates. The plastics may furthermore be fiber-reinforced, in particular using carbon fibers and/or metal fibers.

The substrate resulting from the processes disclosed herein may be used as a component to fabricate vehicles, such as automotive vehicles, including but not limited to automobiles, trucks, and tractors. The substrate may have any shape, but may usually be in the form of automotive body components or automotive interior components.

### Step (i):

In step (i) of the processes disclosed herein, at least part of a surface of the substrate (S) is inoculated with at least one microorganism being capable of producing at least one organic pigment and/or dye. Only part of the surface of the substrate (S) may be inoculated with the at least one microorganism. This may be preferred if only parts of the surface of the substrate are to be colored by organic pigments produced by said microorganism(s). All parts of the surface of the substrate (S) may be inoculated with the at least one microorganism. This may be preferred if the complete surface of the substrate is to be colored by organic pigments produced by said microorganism(s)

In an embodiment, the at least one microorganism is selected from bacteria and/or fungi being capable of producing at least one organic pigment and/or dye, in particular is selected from bacteria being capable of producing at least one organic pigment and/or dye.

Suitable bacteria may include bacteria selected from at least one of Janthinobacterium such as Janthinobacterium such as Janthinobacterium lividum, Chromobacterium such as Chromobacterium violaceum and Chromobacterium sp., Duganella sp., Pseudoalteromonas sp., Collimonas sp., Pseudoalteromonas luteoviolacea, Pseudoalteromonas tunicate, lodobacter, Rugamonas, and Massilia. With particular preference, the microorganism included in aqueous composition (C1) is selected from Janthinobacterium such as Janthinobacterium lividum. Janthinobacterium lividum is commercial available, for example as Janthinobacterium lividum 1522 strain ATCC 12473 from WAAG Society, TextileLab, Amsterdam (Netherlands).

In an embodiment, the at least one organic pigment and/or dye is selected from the group consisting of aromatic and heteroaromatic organic pigments and mixtures thereof. Preferred organic pigments include organic pigments having at least one bis-indole moiety. An exemplary organic pigment having at least one bis-indole moiety is violacein (a purple organic pigment). Violacein may be produced by the aforementioned bacteria.

Further suitable bacteria may be selected from (i) Serratia marcesans, Serratia marcescens, Alteromonas rubra, Streptoverticillium and Pseudoalteromonas rubra (all producing the red organic pigment prodigiosin); (ii) Corynebacterium insidiosum (producing the blue organic pigment indigoidine); (iii) Monascus roseus, Bradyrhizobium sp., Haloferax alexandrines and Lactobacillus pluvialis (all producing the orange/red organic pigment canthaxanthin); (iv) Staphylococcus aureus and Flavobacterium sp. (all producing the yellow organic pigment zeaxanthin); (v) Pseudomonas aeruginosa (producing the green organic pigment pyocyanin blue); (vi) Dunaliella salina and Rhodococcus maris (both producing the orange red organic pigment beta-carotene); (vii) Hematococcus pluvialis, Agrobacterium aurantiacum and Paracoccus carotinifaciens (all producing the red organic pigment astaxanthin); (viii) Asbhya gossypi, Bacillus sp. and Bacillus subtilis (all producing the yellow organic pigment riboflavin); (ix) Blakeslea trispora (producing the red organic pigment lycopene; (x) Rhodotorula sp. (producing the orange/red organic pigment torularhodin); (xi) Penicillium oxalicum and Micromonospora lupine (both producing the red organic pigment anthraquinone); (xii) Streptomyces echinoruber (producing the red organic pigment Rubrolone); (xiii) Streptomyces sp., Pedobacter and Kocuria sp. (producing carotenoids); (xiv) Streptomaces glaucescens (producing the black organic pigment melanin); (xv) Pseudomonas aeruginosa and Pseudomonas guinea (producing the organic pigment pyocyanin); (xvi) Hahella chejuensis (producing the organic pigment prodigines); (xvii), Bacillus cereus (producing the organic pigment azaphenanthrene); (xviii) Chryseobacterium artocarpi (producing the organic pigment flexirubin); (xix) Streptococcus agalactiae (producing the organic pigment granadaene); (xx) Proteobacteria (producing the organic pigment heptyl prodigiosin); (xxi) Corynebacterium insidiosum and Erwinia chrysanthemi (both producing the organic pigment indigoidine); (xxii) Pseudomonas sp. (producing the organic pigment Phenazine); (xxiii) Pseudomonas sp. (producing the organic pigment phycocyanin); (xxiv) Streptomyces echinoruber (producing the organic pigment Rubrolone); (xxv) Staphylococcus aureus (producing the organic pigment staphyloxanthin); (xxvi) Cytophaga/Flexibacteria (producing the organic pigment tryptanthrin); (xxvii) Streptomyces sp. (producing the organic pigment undecyl prodigiosin); (xxviii) Xanthomonas oryzae (producing the organic pigment xanthomonadin); and (xxix) Flavobacterium sp. and Paracoccus sp. (both producing the organic pigment zeaxanthin).

Suitable fungi include (i) stemphylium lycopersici and fusarium oxysporum (both producing the red organic pigment anthraquinone); and (ii) Fusarium sp. (producing the organic pigment benzoquinone).

In an embodiment, inoculating at least part of the surface of the substrate (S) includes applying the at least one microorganism to said surface. For instance, microorganisms grown on a nutrient plate may be applied to the respective substrate surface(s), for example by use of a suitable tool.

In an embodiment, inoculating at least part of the surface of the substrate (S) includes applying an aqueous composition (C1) comprising the at least one microorganism to at least part of the surface of the substrate (S). Said aqueous composition (C1) may further comprise a nutrient medium, for example the nutrient medium used in step (ii) of the processes disclosed herein.

The aqueous composition (C1) may be obtained by preparing a pre-culture and using said preculture to inoculate fresh nutrient medium. Said pre-culture may be prepared by inoculating nutrient medium with the respective microorganism and incubating the nutrient medium at 25°C under agitation. The pre-culture may be incubated until the cell concentration reaches a predefined value, such as an optical density OD₆₀₀ value of 3.

### Step (ii):

In step (ii), at least the part of the inoculated surface is immersed in a nutrient medium such that the at least one microorganism produces the at least one organic pigment and/or dye. The inoculated surface may be completely immersed in the nutrient medium. For instance, the substrate (S) may be totally immersed in the nutrient medium to ensure that all inoculated surfaces of the substrate (S) are sufficiently covered with nutrient medium such that the microorganisms present on said surfaces may produce the organic pigment(s) and/or dyes. The inoculated surface may only be partially immersed in the nutrient medium. For instance, only part of the substrate may be immersed in the nutrient medium. This may be preferred if not all surfaces of the substrate are inoculated with the microorganisms to save nutrient medium or if only part of the surface of the substate is to be colored by pigments produced by microorganisms present on the surface immersed in the nutrient medium.

In an embodiment, the nutrient medium comprises at least one extract, in particular a yeast extract or meat extract, a composition resulting from the at least partial enzymatic or acid hydrolysis of at least one protein, and at least one salt. The composition resulting from the at least partial enzymatic hydrolysis may also be referred to as peptone. The composition of peptone may depend on the protein as well as on the enzymatic source used for digestion. For instance, casein may be used as protein and pancreas extract as enzyme, resulting in a composition called tryptone. In another instance, soy protein may be used as protein and may be digested with the enzyme papain. The nutrient medium may comprise further components such as agar or pH adjusting agents, such as NaOH or HCI. The nutrient medium may consist of the aforementioned components, i.e. the extract(s), the composition resulting from the at least partial enzymatic or acid hydrolysis of at least one protein and the at least one salt.

In an alternative embodiment, the nutrient medium comprises a composition resulting from the at least partial enzymatic or acid hydrolysis of at least one protein, and at least one salt. The nutrient medium may comprise further components such as agar or pH adjusting agents, such as NaOH or HCI. The nutrient medium may consist of the aforementioned components, i.e. the composition resulting from the at least partial enzymatic or acid hydrolysis of at least one protein and the at least one salt.

The at least one salt may be selected from sodium chloride, sodium pyruvate, magnesium sulfate, dipotassium hydrogen phosphate, potassium dihydrogen phosphate, disodium hydrogen phosphate, sodium dihydrogen phosphate, or a combination thereof. With particular preference, the at least one salt is selected from sodium chloride. The at least one salt may be formed in situ by addition of pH adjusting agents, such as NaOH and HCI to adjust the pH of the nutrient medium to the desired pH or pH range.

The at least one extract may be present in a total amount of 2 to 8 g/L nutrient medium. For instance, the at least one extract, such as meat extract or yeast extract, may be present in a total amount of 3 g/L to 5 g/L nutrient medium. The composition resulting from the at least partial enzymatic or acid hydrolysis of at least one protein may be present in a total amount of 3 to 12 g/L nutrient medium. For instance, said composition may be present in a total amount of 5 g/L to 10 g/L nutrient medium. The at least one salt may be present in a total amount of 0.5 to 10 g/L nutrient medium. For instance, the at least one salt may be present in a total amount of 5 g/L nutrient medium.

In an embodiment, the pH at 25°C of the nutrient medium ranges from pH 5 to pH 9. With particular preference, the pH of the nutrient medium at 25°C is pH 7. Use of pH 7 during the immersion of the inoculated substrates resulted in a production of sufficient amounts of organic pigments and/or dyes by the microorganism(s). Use of pH 7 is especially preferred for Janthinobacterium lividum.

Exemplary embodiments of suitable nutrient media are given in the following:
In an embodiment, the nutrient medium contains - per liter nutrient medium - 5 g yeast extract, 10 g tryptone and 5 g sodium chloride and has a pH of 7 at 25°C.

In another embodiment, the nutrient medium contains - per liter nutrient medium - 3 g meat extract, 5 g peptone and 0.5 to 10 g sodium chloride and has a pH of 7 at 25°C.

In an embodiment, immersing at least the part of the inoculated surface in a nutrient medium includes incubating at least the part of the inoculated surface in the nutrient medium at a temperature of 20 to 30°C, in particular at 25°C, for a duration of 3 to 6 days, in particular for 4 days. Incubation of the immersed and inoculated substrate (S) under the aforementioned conditions results in sufficient production of organic pigment and/or dye by the microorganisms present on the surface(s) of the substrate (S) such that a sufficient coloring of the substrate is achieved. The degree of coloring is dependent on the incubation temperature and incubation time. For instance, a lower degree of coloring may be obtained if lower incubation temperatures and/or lower incubation times are used, while a higher degree of coloring may be obtained if higher incubation temperatures and/or prolonged incubation times are used. This allows to adjust the obtained result in step (ii) to the customers needs, thus allowing to provide substrates with different degrees of colorations without having to use a different aqueous composition (C1). This allows to produce highly customized colored substrates from one and the same aqueous composition (C1), rendering the proceses disclosed therein highly efficient and flexible in terms of the achieved substrate coloration.

### Step (iii):

In step (iii) of the processes disclosed herein, the pigment and/or dye producing process is interrupted, i.e. the substrate (S) is treated such that the microorganisms present on the surface of said substrate (S) are no longer capable of producing the organic pigment(s) and/or dye(s).

In an embodiment, interrupting the pigment and/or dye producing process includes
- treating the at least partially colored substrate (S) resulting from step (ii) with steam having a temperature of at least 80°C and at a pressure of more than 1 bar for a duration of 1 to 120 minutes,
- optionally rinsing the treated at least partially colored substrate with an aqueous solution, and/or
- optionally drying the treated or rinsed at least partially coated substrate.

Treating the at least partially colored substrate (S) resulting from step (ii) with steam at elevated pressures may include autoclaving the substrate (S). For instance, the substrate resulting from step (ii) may, for example after incubation of said substrate for 4 days at 25°C in the nutrient medium as described previously, be transferred to an autoclave and autoclaved to interrupt the formation of further organic pigment(s) and/or dye(s) by the microorganisms present on the surface of the substrate (S). Autoclaving may result in killing the microorganisms such that they are no longer capable of producing organic pigment(s) and/or dye(s). This may ensure that the achieved color does not undergo any further changes upon storage of the colored substrate (S) and avoids contamination of the environment of the substrate (S) with living microorganisms being present on said colored substrate (S).

The steam used to treat the at least partially colored substrate may have a temperature of 100°C to 140°C, in particular of 120°C. The pressure used to treat the at least partially colored substrate may range from 1.01 to 2 bar, in particular from 1.01 to 1.3 bar. The at least partially colored substrate may be treated with steam under elevated pressure for a duration of 10 to 60 minutes. The aforementioned conditions ensure that production of further organic pigment(s) and/or dye(s) is effectively prevented such that the color achieved after the end of step (ii) is effectively preserved and the risk of contamination of the environment with microorganisms is effectively avoided.

The treated substrate may be rinsed with water or with an aqueous composition comprising at least one surfactant. The at least one surfactant may be a nonionic surfactant, such as nonionic surfactants having ethylene and/or propylene oxide units. The surfactant may be a cationic surfactant and/or an anionic surfactant. The aqueous composition may contain a mixture of different surfactants, such as anionic surfactants, cationic surfactants and/or nonionic surfactants. Rinsing the treated substate allows to remove residues, such as cell residues resulting from the steam treatment, from the surface of the substrate.

The treated or rinsed substrate may be dried. Drying may be effected by the use of elevated temperatures. Drying may be effected by the use of an air stream. Drying the treated or rinsed substrate ensures a homogenous and uniform distribution of the coating material applied in step (iv) and avoids incompatibilities between the applied coating material and residues resulting from the steam treatment or the rinse process. This allows to obtain a high quality optical appearance of the resulting coated substrate.

### Step (iv):

In step (iv) of the processes disclosed herein, at least one coating material is applied to at least part of the colored substrate obtained after step (iii). The applied coating material(s) may optionally be dried and/or cured. In case more than one coating material is applied in step (iv), the applied coating materials may be jointly cured or may be cured separately, i.e. each applied coating material may be cured prior to application of the subsequent coating material.

Application of at least one coating material to the at least partially colored substrate of resulting after step (iii) of the processes disclosed herein allow to protect the organic pigment(s) and/or dye(s) produced by the microorganisms in step (ii). For instance, application of at least one coating material may prevent degradation of the organic pigment(s) and/or dye(s) by UV irradiation present in daylight, thus avoiding color fading upon exposure of the colored substrate to environmental influences. In another instance, application of at least one coating material may prevent destruction of the substrate (S), for example by environmental influences such as acid rain, stone chipping, etc..

The coating material may be any commonly known coating material used in the state of the art to coat substrates, such as automotive substrates. With preference, the coating material is at least partially transparent, i.e. is not fully opaque, such that the underlying color of the organic pigment(s) and/or dye(s) produced by the mircroorganism(s) is visible. This avoids that the coloration of the substrate achieved by the use of pigment and/or dye producing microorganisms on the substrate is completely hidden by the coating produced from the at least one coating material.

In one embodiment, the coating material is selected from a clearcoat material or a tinted clearcoat material. The clearcoat material may be any transparent coating material, i.e. may not comprise any color pigments, effect pigments, fillers and matting agents. However, the clearcoat material may comprise dyes or transparent pigments not having any visual effect. Tinted clearcoat materials are, when applied to a substrate, neither completely transparent and colorless as a clear coating nor completely opaque as a typical pigmented coating. A tinted clear coating is therefore transparent and colored or semi-transparent and colored. The color may be achieved by adding small amounts of at least one pigment commonly used in coating compositions, for example amounts of 0.1 to 10 wt.-%, preferably 1 to 4 wt.-%, based on the total weight of the tinted clearcoat material.

This clearcoat or tinted clearcoat material may be an aqueous or solvent borne material, which may be formulated either as one-component or two-component material, or multicomponent materials. In case of one-component materials, crosslinking reactions during storage need to be avoided, for example by using blocked crosslinking agents which are only reactive at higher temperatures. Two-component materials comprise the reactive components, i.e. binder and crosslinker, in separate containers. The components of containers 1 and 2 are then mixed prior to use, preferably shortly before application of the mixed material to the substrate. In addition, powder slurry clearcoat or tinted clearcoat materials are also suitable.

The clearcoat or tinted clearcoat material may be a thermally and/or chemically curing clearcoat or tinted clearcoat material. Such materials may comprise at least one (first) polymer as a binder having functional groups, and at least one crosslinker having a functionality complementary to the functional groups of the binder. Preference is given to using at least one hydroxy-functional poly(meth)acrylate polymer as a binder and a polyisocyanate as a crosslinking agent. Suitable clearcoat materials are described, for example, in WO 2006/042585 A1, WO 2009/077182 A1 or else WO 2008/074490 A1.

In an embodiment, the at least one coating material is a radiation curable coating material. Examples of suitable radiation sources are low-pressure, medium-pressure, and high-pressure mercury emitters, fluorescent tubes, pulsed emitters, metal halide emitters (halogen lamps), lasers, LEDs, and also electronic flash installations or excimer emitters. It is of course also possible to use two or more radiation sources - two to four, for example. These sources may also each emit in different wavelength ranges. With particular preference, a UV radiation source is used in step (2) of the inventive process.

Electron beam curing is usually effected with an electron accelerator. Individual accelerators are usefully characterized by their energy, power, and type. Low-energy accelerators provide beam energies from about 150 keV to about 2.0 MeV. Medium-energy accelerators provide beam energies from about 2.5 to about 8.0 MeV. High-energy accelerators provide beam energies greater than about 9.0 MeV. Accelerator power is a product of electron energy and beam current. Such powers range from about 5 to about 300 kW. The main types of accelerators are: electrostatic direct-current (DC), electrodynamic DC, radiofrequency (RF) linear accelerators (LINACS), magnetic-induction LINACs, and continuous-wave (CW) machines.

Suitable UV curable coating materials are disclosed, for example, in unpublished international patent application PCT/EP2022/065053. Said coating materials are especially suitable for plastic substrates and result in coatings having a high adhesion to the underlying substrate without any pretreatment steps as well as a high flexibility.

The coating material may be applied by methods known to those skilled in the art for application of liquid coating compositions, for example by dipping, bar coating, spraying, rolling or the like. Preference is given to employing spray application methods, for example compressed air spraying (pneumatic application), and electrostatic spray application (ESTA).

The applied coating material may be dried and/or cured. Drying of the applied coating material may include thermal drying and/or drying by irradiation, for example if the coating material is a UV-curable coating material. Thermal drying may include subjecting the applied coating material to a temperature of 20 to 60 °C for a duration of 5 to 80 minutes, preferably to a temperature of 20 to 35 °C for a duration of 5 minutes to 70 minutes. Drying by irradiation may include subjecting the applied coating material to an intensity of 100 to 300 mJ/cm², in particular to an intensity of 150 to 210 mJ/cm².

The intensity used for UV-curing may range from 100 to 1,000 mJ/cm². It may be preferred if the curing is performed in two steps with different intensities, namely a first intensity of 160 to 210 mJ/cm² followed by second intensity of 750 to 1,000 mJ/cm². Performing the curing in two steps with different intensities may be beneficial with respect to the degassing of the coating material during curing and the adhesion of the cured coating film to the underlying substrate.

The dry film thickness of the resulting coating layer (i.e. the dry film thickness of the cured coating material) may vary greatly and primarily depends on the surface roughness of the substrate. It may range, for example, from 20 to 200 µm, preferably from 20 to 100 µm. Film thicknesses of up to 100 or 200 µm may be obtained by repeating step (iv) at least once, in particular at least twice.

### At least partially colored substrate (S):

A second subject-matter of the present disclosure is an at least partially colored substrate (S) produced by the processes disclosed herein. The at least partially colored substrate is obtained using sustainable organic pigment(s) and/or dye(s) by incubating microorganism(s) producing such pigment(s) and/or dye(s) with the substrate in a nutrient medium.

What has been said about the processes described herein applies mutatis mutandis with respect to further preferred embodiments of the at least partially coated substrate (S).

### EXAMPLES

The present invention will now be explained in greater detail by the use of working examples, but the present invention is in no way limited to these working examples. Moreover, the terms "parts", "%" and "ratio" in the examples denote "parts by mass", "mass %" and "mass ratio" respectively unless otherwise indicated.

### 1. Preparation of substrates

The following substrates were used:
- car shaped polyamide substrates obtained from 3D printing processes using Ultramide^{®} advanced N4H,
- Steel panels (30 x 50 cm, Chemetall) coated with a cured standard cathodic electrocoat material (Cathoguard 800, available from BASF Coatings GmbH),
- polyamide 6 (PA 6), polyamide 11 (PA 11), polyamide 12 (PA 12) and polypropylene panels.

### 2. Inoculation of substrates

Nutrient broth (NB) consisting of 3 g/L meat extract, 5 g/L peptone and 5 g/L salts, such as sodium chloride, was used as a medium for bacterial cultures. The pH of the NB was set to 7 (at 25°C). Nutrient agar petri-dishes were prepared with composition 3 g/L meat extract, 5 g/L peptone, and 5 g/L agar.

*Janthinobacterium lividum* (*J. lividum*) was purchased commercially. For example, *Janthinobacterium lividum* 1522 strain ATCC 12473 can be purchased from WAAG Society, TextileLab, Amsterdam (Netherlands) and may be maintained frozen in Luria Bertani (LB) broth containing glycerol 20% (v/v) at -80°C.

### 2.1 Inoculation with grown bacteria

Nutrient agar petri-dishes were prepared prior to their inoculation with *J. lividum* stock at -80°C and were incubated at 25°C overnight. The grown culture was applied to at least part of the surface of the substrates.

### 2.2 Inoculation with aqueous composition (C1)

The aqueous composition (C1) may be obtained by preparing a pre-culture and using said preculture to inoculate fresh nutrient medium. Said pre-culture may be prepared by inoculating nutrient medium with the respective microorganism and incubating the nutrient medium at 25°C under agitation. The pre-culture may be incubated until the cell concentration reaches a predefined value, such as an optical density OD₆₀₀ value of 3. The aqueous composition (C1) was applied to at least part of the surface of the substrates.

### 3. Incubation of substrates with microorganisms capable of producing pigment/dye

The respective substrates, the incubator as well as the NB were steam sterilized in an autoclave at 120 °C for around 20 minutes.

After sterilization, at least part of the surface of the substrates is inoculated with grown bacteria prepared according to point 2.1 or with the aqueous composition (C1) prepared in point 2.2.

The inoculated substrates were immersed in NB at 25°C for 4 days in the incubator such that *J. lividum* present on the surface produce the organic pigment violacein.

Afterwards, the at least partially colored substrates were steam sterilized in an autoclave to stop production of the organic pigment violacein by killing the *J. lividum* present on the surface. The sterilized colored substrates were washed with water and dried with compressed air.

### 4. Preparation of coated substrates

A clearcoat material was prepared by mixing the ingredients listed in Table 1. The clearcoat material had a viscosity of 10 seconds at 23 °C (determined according to DIN EN ISO 2431:2020-02 using a DIN 6 cup) and a solid content of at least 74 wt.% (determined according to DIN EN ISO 3251: 2018-07 at 130°C; 60 min, starting weight 1.0 g).

**Table 1: Ingredients for the clearcoat material (all amounts are given in wt.%, based on the total weight of the clearcoat material)**

| | Amount [wt.%] |
|---|---|
| Ebecryl 5129 ¹⁾ | 12.9 |
| Ebecryl 767 ²⁾ | 22.4 |
| Laromer DPGDA ³⁾ | 14.9 |
| Butandiol monoacrylate | 14.9 |
| Isobornyl acrylate | 14.9 |
| Hexandiol diacrylate | 4.40 |
| Omnirad 754 ⁴⁾ | 5.20 |
| Ethyl acetate | 10.4 |

| | |
|---|---|
| ¹⁾ hexafunctional aliphatic urethane acrylate oligomer (60wt.% in 40wt.% of a mixture of pentaerythritol triacrylate and pentaerythritol tetracrylate; supplied by Allnex GmbH) ²⁾ acid functionalized acrylic resin diluted with 38 wt.% of isobornyl acrylate monomer and 2 wt.% of trimethylolpropane triacrylate (supplied by Allnex GmbH) ³⁾ dipropylenglycol diacrylate, calculated Hansen solubility parameter δₚ = 6.2 MPa^{1/2} (supplied by BASF SE) ⁴⁾ blend of oxy-phenyl-acetic acid 2-[2-oxo-2-phenyl-acetoxy-ethoxy]-ethyl ester and oxy-phenyl-acetic acid 2-[2-hydroxy-ethoxy]-ethyl ester (supplied by IGM Resins) | |

The clearcoat material prepared as described above was applied to the dried and colored substrates obtained after point 3. by pneumatic spray application at 23 °C and 63% relative humidity using a pressure of 1.3 bar (spray gun: SATA minjet 4400B RP Digital, nozzle size: 0.8-1 mm) such that the resulting dry film thickness is 25 µm. Afterwards, the applied coating layer was dried using a UV light source (commercially available mercury lamp having UVA, UVB und UVC light sources) at an intensity of 170 to 190 mJ/cm² (measured with UV radiometer IL390 or IL490-UVA) and cured with the UV light source at an intensity of 170 to 190 mJ/cm² followed by an intensity of 800 to 950 mJ/cm².

### 5. Properties of the colored substrates

After storage of the colored substrates resulting after point 4. in daylight, no fading of the organic pigment violacein was observed. Use of a clearcoat layer thus results in a sufficient UV stability of the colored substrates.

## Claims

1. A process for producing an at least partially colored substrate (S), said method comprising
(i) inoculating at least part of a surface of the substrate (S) with at least one microorganism being capable of producing at least one organic pigment and/or dye,
(ii) immersing at least the part of the inoculated surface in a nutrient medium such that the at least one microorganism produces the at least one organic pigment and/or dye,
(iii) interrupting the pigment and/or dye producing process,
(iv) applying at least one coating material to at least part of the colored substrate and optionally drying and/or curing the applied coating material(s).

2. The process according to claim 1, wherein the substrate (S) is selected from plastics substrates, metal substrates and substrates containing plastic and metal parts.

3. The process according to claim 1 or 2, wherein the at least one microorganism is selected from bacteria and fungi being capable of producing at least one organic pigment and/or dye.

4. The process according to claim 3, wherein the bacteria are selected from at least one of Janthinobacterium such as Janthinobacterium such as Janthinobacterium lividum, Chromobacterium such as Chromobacterium violaceum and Chromobacterium sp., Duganella sp., Pseudoalteromonas sp., Collimonas sp., Pseudoalteromonas luteoviolacea, Pseudoalteromonas tunicate, lodobacter, Rugamonas, and Massilia, preferably from Janthinobacterium such as Janthinobacterium lividum.

5. The process according to one or more of the preceding claims, wherein the at least one organic pigment and/or dye is selected from the group consisting of aromatic and heteroaromatic organic pigments and mixtures thereof, in particular organic pigments having at least one bis-indole moiety such as violacein.

6. The process according to any one of the preceding claims, wherein inoculating at least part of the surface of the substrate (S) includes applying the at least one microorganism to said surface; and/or wherein inoculating at least part of the surface of the substrate (S) includes applying an aqueous composition (C1) comprising the at least one microorganism to at least part of the surface of the substrate (S).

7. The process according to any one of the preceding claims, wherein the nutrient medium comprises at least one extract, in particular a yeast extract or meat extract, a composition resulting from the at least partial enzymatic or acid hydrolysis of at least one protein, and at least one salt and/or wherein the pH at 25°C of the nutrient medium ranges from pH 5 to pH 9, in particular is pH 7.

8. The process according to claim 7, wherein the at least one salt is selected from sodium chloride, sodium pyruvate, magnesium sulfate, dipotassium hydrogen phosphate, potassium dihydrogen phosphate, disodium hydrogen phosphate, sodium dihydrogen phosphate, or a combination thereof.

9. The process according to claim 7 or 8, wherein the at least one extract is present in a total amount of 2 to 8 g/L nutrient medium, and/or wherein the composition resulting from the at least partial enzymatic or acid hydrolysis of at least one protein is present in a total amount of 3 to 12 g/L nutrient medium, and/or wherein the at least one salt is present in a total amount of 0.5 to 10 g/L nutrient medium.

10. The process according to one or more of the preceding claims, wherein immersing at least the part of the inoculated surface in a nutrient medium includes incubating at least the part of the inoculated surface in the nutrient medium at a temperature of 20 to 30°C, in particular at 25°C, for a duration of 3 to 6 days, in particular for 4 days.

11. The process according to one or more of the preceding claims, wherein interrupting the pigment and/or dye producing process includes
- treating the at least partially colored substrate (S) resulting from step (ii) with steam having a temperature of at least 80°C and at a pressure of more than 1 bar for a duration of 1 to 120 minutes,
- optionally rinsing the treated at least partially colored substrate with an aqueous solution, and/or
- optionally drying the treated or rinsed at least partially coated substrate.

12. The process according to claim 11, wherein the steam has a temperature of 100°C to 140°C, in particular of 120°C, and/or wherein the pressure is from 1.01 to 2 bar, in particular from 1.01 to 1.3 bar, and/or wherein the duration is from 10 to 60 minutes.

13. The process according to one or more of the preceding claims, wherein the at least one coating material is selected from a clearcoat material or a tinted clearcoat material.

14. The process according to one or more of the preceding claims, wherein the at least one coating material is a radiation curable coating material.

15. At least partially colored substrate (S) produced by a process as claimed according to any of claims 1 to 14.
